# EUROPEAN PATENT SPECIFICATION

(11) **EP 1 436 399 B1**
(45) Date of publication and mention of the grant of the patent: **18.02.2009**
(21) Application number: 02768878.7
(22) Date of filing: 19.09.2002
(51) Int. Cl.: C12N 15/63, C12N 5/00, C12N 15/09, A01K 67/00

(54) **ANIMAL INTEGRATION VECTOR AND METHODS FOR ITS USE**
TIERISCHER INTEGRATIONSVEKTOR UND VERFAHREN FÜR SEINE VERWENDUNG
VECTEUR D'INTEGRATION ANIMALE ET SES PROCEDES D'UTILISATION

(30) Priority: 24.09.2001 US 962853
(43) Date of publication of application: 14.07.2004
(73) Proprietor: Tosk, Inc., Santa Cruz, CA 95060 (US)
(72) Inventor: FOGARTY, Patrick, Santa Cruz, CA 95060 (US)
(74) Representative: Brasnett, Adrian Hugh
(86) International application number: PCT/US2002/029999
(87) International publication number: WO 2003/027241

(56) References cited:
- WO-A1-99/09817
- US-A- 5 719 055
- US-B1- 6 291 243
- BEALL E.L. ET AL.: 'Drosophila P-element transposase is a novel site-specific endonuclease' GENES & DEVELOPMENT vol. 11, 1997, pages 2137 - 2151, XP002962731
- RAZ E; VAN LUENEN H G; SCHAERRINGER B; PLASTERK R H; DRIEVER W: "Transposition of the nematode Caenorhabditis elegans Tc3 element in the zebrafish Danio rerio" CURRENT BIOLOGY, vol. 8, no. 2, 15 January 1998 (1998-01-15), pages 82-88, XP000700202
- KHILLAN J S; OVERBEEK P A; WESTPHAL H: "Drosophila P element integration in the mouse." DEVELOPMENTAL BIOLOGY, vol. 109, no. 1, 1985, pages 247-250, XP009082327

## Description

### Field of the Invention

The field of this invention is nucleic acid vectors.

### Background of the Invention

Delivery of nucleic acids into a cell in culture or a whole animal is a process known as transformation. Transformation plays a major role in a variety of biotechnology and related applications, including research, synthetic and therapeutic applications. Research applications where transformation in cultured cells plays a critical role include the production of recombinant bacteria and yeast for cloning applications. Likewise, the production of transgenic animals has provided invaluable knowledge into gene function, especially C. elegans and D. melanogaster. Synthetic applications in which transformation plays a critical role include the production of peptides and proteins, where both animal and cellular systems are employed. Therapeutic applications in which transformation plays a key role include gene therapy applications, where both cellular and animal systems are employed. Because of the prevalent role transformation plays in the above and other applications, a variety of different transformation protocols have been developed.

In many transformation applications, it is desirable to introduce the exogenous DNA in a manner such that it is incorporated into a target cell's genome. One means of providing for genome integration is to employ a vector that is capable of homologous recombination. This technique is limited due to low efficiency and is applicable for cellular use only where the desired transformation event can be selected from a pool of 1000's of possibilities.

Another approach uses viral based transformation to integrate nucleic acids into the genome. Viral vectors are limited in their efficiency by the size of the nucleic acid to be delivered. Also, these viruses are limited to the species and cell type with which they can deliver nucleic acids. These limitations are for both cells and whole animals, but whole animals have further limitations. Viruses are efficiently eliminated from an animal before they are able to deliver nucleic acids. Further, if gene expression is desired from a delivered nucleic acid, this too can be problematic when using a virus to deliver into a whole animal. Many times, even if the virus successfully avoids the animal's immune response and delivers the nucleic acid into the genome, cellular mechanisms shut off the gene expression due to intracellular viral detection. Viral based vectors finding use include retroviral vectors, e.g., Maloney murine leukemia viral based vectors. Other viral based vectors that find use include adenovirus derived vectors, HSV derived vectors, sindbis derived vectors, etc. While viral vectors provide for a number of advantages, their use is not optimal in many situations.

More effective transformation vectors are continually being produced, but these are primarily for cellular transformation, primarily because of the dramatic difference in complexity when operating in a cell versus a whole animal. Accordingly, transformation vectors that integrate nucleic acids into the genome for whole animal systems are rare.

Therefore, there is continued interest in the development of new transformation vectors. Of particular interest is the development of non-viral vectors that provide for stable integration of exogenous DNA into a cellular genome in an intact animal.

### Relevant Literature

U.S. Patents of interest include: 5,719,055 and 4,670,388. Other references of interest include: Beall et al, Drosophila P element transposase is a novel site-specific endonuclease, Genes and Development, vol.11, 2137-2151 (1997).; Ivics et al. (1996). "Identification of functional domains and evolution of Tc1-like transportable elements" Proc. Natl. Acad. Sci., vol. 93: 5008-5013; Ivics et al. (1997). "Molecular Reconstruction of Sleeping Beauty, a Tc1-like Transposon from Fish, and its Transposition in Human Cells" Cell, vol. 91: 501-510; Luo et al. (1998). "Chromosomal transposition of a Tc1/mariner-like element in mouse embryonic stem cells" Proc. Natl, Acad. Sci., vol. 95: 10769-10773; Rio et al. (1986). "Identification and Immunochemical Analysis of Biologically Active Drosophila P Element Transposase" Cell, Vol. 44: 21-32; Rio et al. (1988). "Evidence for Drosophila P Element Transposase Activity in Mammalian Cells and Yeast" J. Mol. Biol., vol. 200: 411-415; Schouten et al. (1998). "Transposon Tc1 of the nematode Caenorhabditis elegans jumps in human cells" Nucleic Acid Research, vol. 26(12): 3013-3017; and Rio, D.C. "Regulation of Drosophilia P element transposition", Trends in Genetics, Sep. 1991, vol. 7, No. 9, pp. 282-287. US 6,291,243 describes a vector comprising a pair of P element transposase recognised insertion sequences flanking at least two transcriptionally active genes, wherein the vector may or may not include transcription regulatory elements therefor.

### SUMMARY OF THE INVENTION

The invention relates to an animal integration vector and methods for its use in the insertion of an exogenous nucleic acid into the genome of a whole animal are provided. The vectors of the subject invention include a pair of transposase recognized insertion sequences, e.g., P element transposase recognized insertion sequences (such as P element derived 31 base pair inverted repeats), flanking at least one transcriptionally active gene that is located sufficienty proximal to one of the transposase recognized insertion sequences, e.g., P-feet, to provide for the desired genome integration. In practicing the subject methods, a vector as described above carrying an exogenous or endogenous nucleic acid is introduced into a target animal under conditions sufficient for entry into the animal and its cells resulting in transposition of the exogenous nucleic acid from the vector into the animal genome, i.e., in the genome of at least a portion of the cells making up the animal. The subject methods find use in a variety of transformation applications, including research, polypeptide synthesis and therapeutic applications.

In particular, the present invention provides a P-element vector that integrates into the genome of a non-Drosophilidae animal, said vector comprising: a pair of P-element transposase recognized insertion sequences flanking one transcriptionally active gene that is located in sufficient proximity to at least one of the P-element transposase recognised insertion sequences to provide for the animal genome integration, wherein the transcriptionally active gene includes a coding sequence of nucleotides in operational combination with a promoter and optionally further comprising a transposase domain encoding a product having P-element transposase activity wherein said transposase domain is outside of the domain flanked by said pair of P-element transposase recognised insertion sequences.

The invention also provides:
the use of a P-element vector as defined above in the manufacture of a composition for insertion of a transcriptionally active gene into the genome of a non-Drosopholidae non-human animal wherein said vector is introduced into said animal under conditions sufficient for transposition to occur so that said transcriptionally active gene is inserted into said genome; and
the use of a P-element vector as defined in above wherein said transcriptionally active gene is a therapeutic gene in the manufacture of a gene therapy vector for insertion of a transcriptionally active gene into the genome of a non-Drosopholidae animal wherein said vector is introduced into said animal under conditions sufficient for transposition to occur so that said transcriptionally active gene is inserted into said genome.

The invention further provides:
a kit for *in vitro* use in inserting an exogenous nucleic acid into the genome of a target animal cell, said kit comprising a P-element vector of the invention and a nucleic acid sequence encoding a product having P-element transposase activity;
a kit for use in inserting an exogenous nucleic acid into the genome of a target non-human animal cell, said kit comprising a P-element vector a P-element vector of the invention and a nucleic acid sequence encoding a product having P-element transposase activity; and
a kit for use in inserting an exogenous nucleic acid into the genome of a target animal cell, said kit comprising a P-element vector of the invention wherein the transcriptionally active gene is a therapeutic gene; and a nucleic acid sequence encoding a product having P-element transposase activity.

### BRIEF DESCRIPTION OF THE DRAWINGS

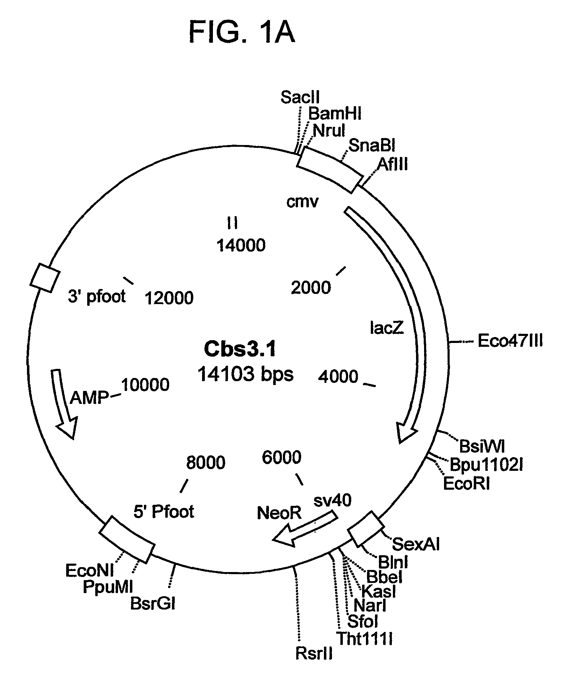

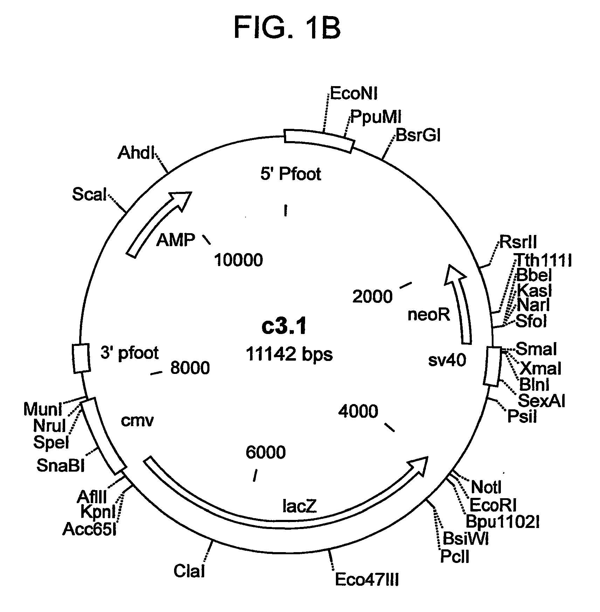

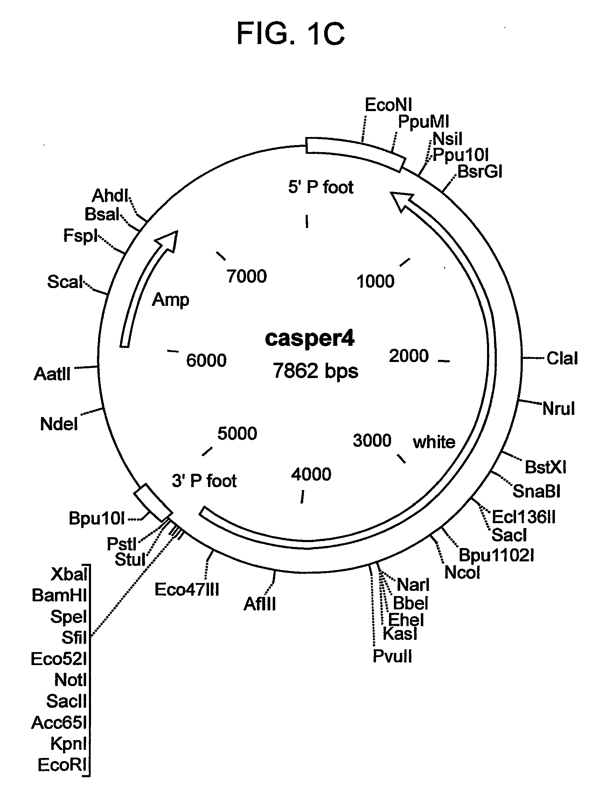

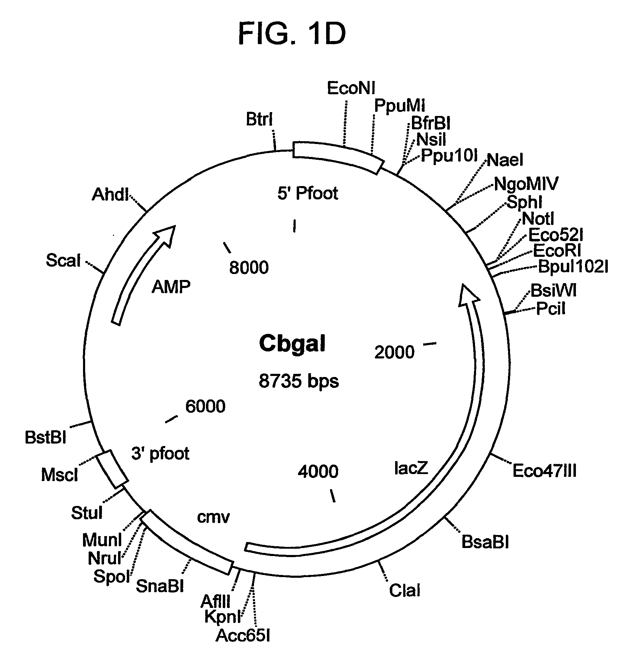

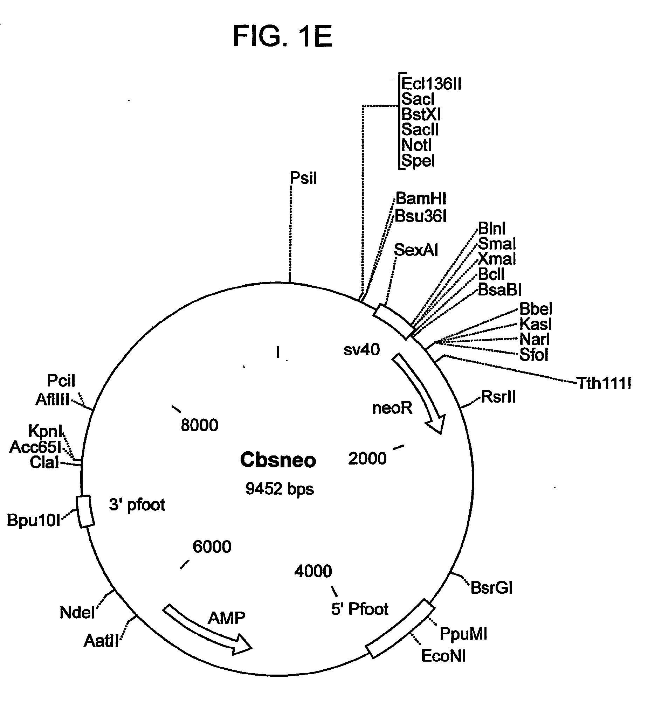

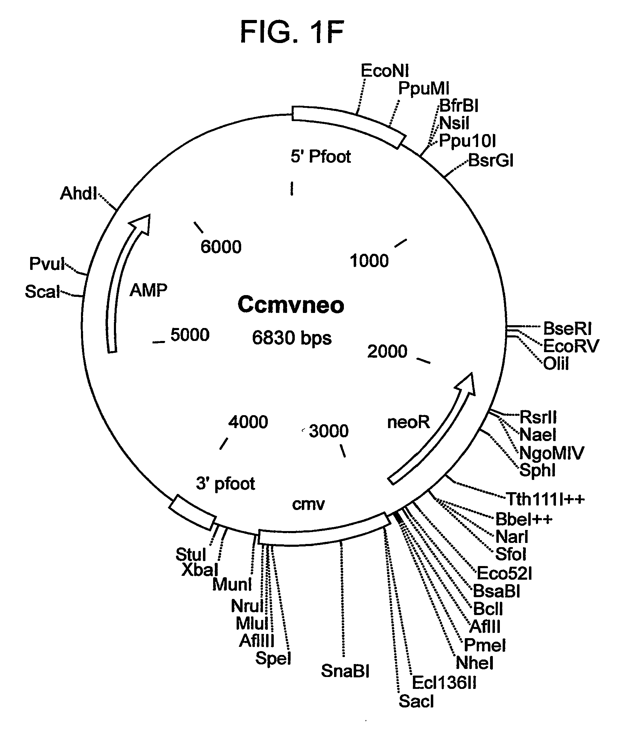

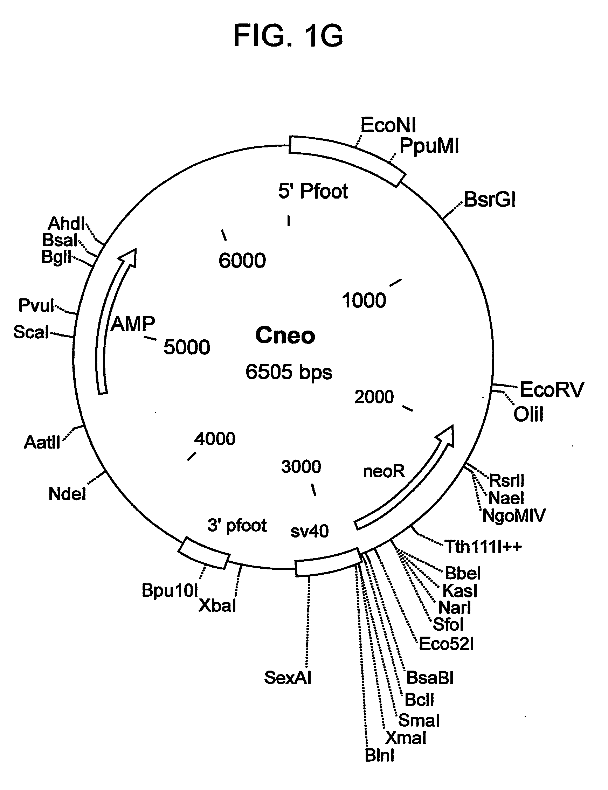

Figs. 1A to 1G provide diagrams of the vectors recited in the Experimental Section, below.

### DESCRIPTION OF THE SPECIFIC EMBODIMENTS

Animal integration vectors and methods for their use in the insertion of an exogenous nucleic acid into the genome of a whole animal, i.e., into the genomic DNA of at least a portion of the cells that make up the animal, are provided. The subject vectors include two transposase recognized insertion sequences, e.g. P element transposase recognized insertion sequences (such as P element derived 31 base pair inverted repeats), flanking at least one transcriptionally active gene that is located in sufficient proximity to at least one of the transposase recognized insertion sequences to provide for the animal genome integration. In the subject methods, a vector according to the invention that may include an exogenous nucleic acid is administered to a whole animal under conditions sufficient for integration of the vector into the animal genome to occur. The subject methods find use in a variety of different applications, including gene therapy applications. In further describing the subject invention, the subject vectors will be described first, followed by a discussion of the methods of using the subject vectors for transformation of a target cell.

In this specification and the appended claims, the singular forms "a," "an" and "the" include plural reference unless the context clearly dictates otherwise. Conversely, it is contemplated that the claims may be so-drafted to exclude any optional element. This statement is intended to serve as antecedent basis for use of such exclusive terminology as "solely," "only" and the like in connection with the recitation of claim elements or by use of a "negative" limitation

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention. Also, it is contemplated that any optional feature of the inventive variations described herein may be set forth and claimed independently, or in combination with any one or more of the features described herein.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

### VECTORS

In the subject vectors, a pair of transposase recognized insertion sequences (e.g., P feet (as described in greater detail below)) flank at least one transcriptionally active gene that is in approximation to, i.e., is sufficientyly close to or sufficiently proximal to, at least one of the transposase recognized insertion sequences so as to provide for the desired genomic integration. By at least one is meant one or more, usually no more than five, and more usually no more than four, where the number of transcriptionally active genes in the vector is often one, two, or three, where only one of the transcriptionally active genes need be sufficiently proximal to the transposase recognized insertion sequence. By in approximation to, i.e., sufficiently close or proximal to, is meant that the transcriptionally active gene is located at a distance from one of the transposase recognized insertion sequences that typically is less than about 7000 bp and often less than about 6,000; 5,000; 4,000; 3,000; or 2,000 bp, where in many embodiments the distance separating the transcriptionally active gene from the transposase recognized insertion sequences does not exceed about 1,000 bp. In certain embodiments, the exogenous nucleic acid that is inserted into the genome of a whole animal in the subject methods, described in greater detail *infra,* is one of the transcriptionally active genes of the vectors.

As indicated above, the vectors of the subject invention include a pair of transpoase recognized insertion sequences flanking at least one transcriptionally active gene or coding sequence. The transposase recognized insertion sequences may be recognized by a number of different transposases. Eukaryotic transposases have the ability to recombine DNA at specific recombination sequences, thereby excising the intervening DNA. See, Haren L, et al., Annu Rev Microbiol 53:245-81 (1999); Hallet B, et al., FEMS Microbiol Rev. 21(2):157-78 (1997). Transposases of interest include, e.g., the maize AC *transposase* (Haring, et al., Plant Mol Biol 16(3):449-61 (1991)), Drosophila P elements (Lankenau, Chromosoma 103(10):659-68 (1995)), insect Tc1/mariner transposons (Plasterk, Curr. Top. Microbiol. Immunol. 204:125-43 (1996); Plasterk, et al., Trends Genet 15(8):326-32 (1999)), including the fish *sleeping* beauty transposon (Ivics, Cell 91(4):501-10 (1997)) and yeast Ty elements (Kim, Genome Res 8(5):464-78 (1998)). Representative specific transposases of interest include, but are not limited, to: transpoases of the TC1 mariner family, e.g., Sleeping Beauty, etc.; the P-element transposase; Piggyback, TN9; and the like.

In certain embodiments of interest, the transpoase recognized insertion sequences are recognized by the P element transposase. In these embodiments, the vectors of the subject invention are P element containing vectors, i.e., P element containing transposon vectors, by which is meant that the vectors include at least the P element transposase recognized insertion sequences of the *Drosophila* P element. As such, the subject vectors include a pair of the 31 base pair inverted repeat domain of the P element, or the functional equivalent thereof, i.e., a domain recognized by the P element encoded transposase. The 31 base pair inverted repeat (i.e. P foot) is disclosed in Beall et al., "Drosophila P-element transposase is a novel site-specific endonuclease," Genes Dev (Aug 15, 1997)11(16):2137-51.

By transcriptionally active gene is meant a sequence that is at least capable of binding the transcriptional factors that initiate the transcription of DNA sequences into RNA sequences under intracellular conditions, e.g., any requisite expression regulatory elements that are required for expression in the intracellular environment. As such, the transcriptionally active genes of the subject vectors typically include a stretch of nucleotides or domain, i.e., expression module, that includes a coding sequence of nucleotides in operational combination, i.e., operably linked, with requisite trascriptional mediation or regulatory element(s). Requisite transcriptional mediation elements that may be present in the expression module include promoters, enhancers, termination and polyadenylation signal elements, splicing signal elements, and the like. In many embodiments, the transcriptionally active gene or expression module is also correctly referred to as an expression cassette.

Preferably, the expression module includes transcription regulatory elements that provide for expression of the gene in a broad host range. A variety of such combinations are known, where specific transcription regulatory elements include: SV40 elements, as described in Dijkema et al., EMBO J. (1985) 4:761; transcription regulatory elements derived from the LTR of the Rous sarcoma virus, as described in Gorman et al., Proc. Nat'l Acad. Sci USA (1982) 79:6777; transcription regulatory elements derived from the LTR of human cytomegalovirus (CMV), as described in Boshart et al., Cell (1985) 41:521; hsp70 promoters, (Levy-Holtzman ,R. and I. Schechter (Biochim. Biophys. Acta (1995) 1263: 96-98) Presnail, J.K. and M.A. Hoy, (Exp. Appl. Acarol. (1994) 18: 301-308)) and the like. In addition, the subject vectors typically include at least one restriction endonuclease recognized site. e.g., restriction site, located between the transposase recognized insertion sequences which serves as a site for insertion of an exogenous nucleic acid. A variety of restriction sites are known in the art and may be included into the vector, where such sites include those recognized by the following restriction enzymes: *Hind*III, *Pst*I*, Sal*I*, Acc*I*, Hinc*II*, Xba*I*, Bam*HI*, Sma*I*, Xma*I*, Kpn*I*, Sac*I, *Eco*RI*,* and the like. In many embodiments, the vector includes a polylinker, i.e., a closely arranged series or array of sites recognized by a plurality of different restriction enzymes, such as those listed above.

The inter transposase recognized insertion sequencedomain of the vectors (i.e., the space between the transposase recognized insertion sequences or that domain or region of the vector located or positioned between the transposase recognized insertion sequence (where this domain includes at least one transcriptionally active gene and the exogenous nucleic acid, when present), may vary greatly in size. Typically, the size of this inter transposase recognized insertion sequencedomain (e.g., P feet flanked domain) is at least about 50 bp in length, usually at least about 1,000 bp in length and more usually at least about 2,000 bp in length, where the length of this domain may be as long as 150,000 bp or longer, but sometimes does not exceed about 50,000 bp in length and in certain embodiments does not exceed about 20,000 bp in length.

In certain embodiments, the subject vectors further include a transposase encoding domain, i.e., a region of nucleotides having a sequence that encodes a protein having transposase activity, particularly a transposase activity that recognizes the transposase recognized insertion sequence being used, e..g,, the P feet of the P element. Transposases of ' interest include, but are not limited to, the specific representative transposases listed above.

Incertain embodiments the protein is a protein having the P element transposase activity, i.e., P element transposase or a functional equivalent or mimetic thereof. The amino acid sequence of the P element transposase is disclosed in Rio et al., Cell (January 17, 1986) 44: 21-32. A specific transposase encoding nucleic acid that may be present on the subject vectors is that found in pTURBO, where the sequence of this plasmid is disclosed in W.R. Engels, Bioess. 14: 681-686 (1992); and at the URL created by placing http:// in front of "firefly.bio.indiana: and .edu at then end of this term. When present on the subject vectors, this P element transposase encoding region or domain is located outside the region flanked by the P feet. In other words, the transposase encoding region is located external to the region flanked by the P feet, i.e., outside the inter P-feet domain described *supra.* Put another way, the tranposase encoding region is positioned to the left of the left terminal P foot or the right of the right terminal P foot.

The subject vectors can be used to stably insert a wide variety of endogenous and/or exogenous nucleic acids into the genome of a whole animal (exogenous means a nucleic acid having a sequence that is not present in the target cell while endogenous means a nucleic acid that pre-exists in the target cell, prior to insertion). By genome of a whole animal is meant that the nucleic acid is inserted or integrated into the genomic DNA of at least a portion of the cells that make up the whole animal, e.g., at least about 5 number %, often at least about 10 number % and sometimes at least about 25 number % or more, including at least about 30, 40, 50, 60, 70, 80, 90, 95, and 99 number % or more of the cells in the whole animal or targeted organ(s) and/or tissue(s). In many embodiments, the individual cells of the animal into which the nucleic acid is integrated are distributed over a wide range of tissue types, e.g., at least about 5 different and often at least about 10 different tissue types, but may be targeted to one specific tissue or organ type. The nature of the nucleic acid will vary depending the particular protocol being performed. For example, in research applications, the exogenous or endogenous nucleic acid may be a novel gene whose protein product is not well characterized. In such applications, the vector is employed to stably introduce the gene into the genome of the target non-human whole animal and observe changes in the phenotype in order to characterize the gene. Alternatively, in protein synthesis application, the exogenous or endogenous nucleic acid encodes a protein of interest which is to be produced by the non-human animal. In yet other embodiments where the vector is employed as a gene therapy vector, the exogenous or endogenous nucleic acid is a gene having therapeutic activity, i.e. a gene that encodes a product of therapeutic utility. The nucleic acid may vary greatly in size. Generally, the size of the nucleic acid that is carried by the vector is at least about 50 bp, usually at least about 1000 bp and more usually at least about 2000 bp, where the length may be as long as 150,000 bp or longer. In many embodiments, the exogenous nucleic acid is long, by which is meant that it ranges in length from about 30 to 50 kb.

The subject vectors may further comprise one or more elements required for amplification of the vector in a prokaryotic host, e.g. *E. coli.* Elements that may be included on the vector for use in amplification of the vector in a prokaryotic host include: an origin of replication, a selectable marker, and the like.

### METHODS OF PREPARING THE SUBJECT VECTORS

The vectors of the subject invention may be produced by standard methods of restriction enzyme cleavage, ligation and molecular cloning. One protocol for constructing the subject vectors includes the following steps. First, purified nucleic acid fragments containing desired component nucleotide sequences as well as extraneous sequences are cleaved with restriction endonucleases from initial sources, e.g., the *Drosophila* P element. Fragments containing the desired nucleotide sequences are then separated from unwanted fragments of different size using conventional separation methods, e.g., by agarose gel electrophoresis. The desired fragments are excised from the gel and ligated together in the appropriate configuration so that a circular nucleic acid or plasmid containing the desired sequences, e.g. sequences corresponding to the various elements of the subject vectors, as described above is produced. Where desired, the circular molecules so constructed are then amplified in a prokaryotic host, e.g. *E. coli.* The procedures of cleavage, plasmid construction, cell transformation and plasmid production involved in these steps are well known to one skilled in the art and the enzymes required for restriction and ligation are available commercially. (See, for example, R. Wu, Ed., Methods in Enzymology, Vol. 68, Academic Press, N.Y. (1979); T. Maniatis, E. F. Fritsch and J. Sambrook, Molecular Cloning: A Laboratory Manual, Cold Spring Harbor Laboratory Press, Cold Spring Harbor, N.Y. (1982); Catalog 1982-83, New England Biolabs, Inc.; Catalog 1982-83, Bethesda Research Laboratories, Inc. An example of how to construct a vector of the present invention is provided in the Experimental section, *infra,* and depicted in Figure 2.

### METHODS OF USING THE SUBJECT VECTORS

The subject vectors find use in a variety of applications in which it is desired to introduce and stably integrate an exogenous or endogenous nucleic acid into the genome of a whole animal. As mentioned above, exogenous nucleic acid means a stretch of nucleotides that is not initially present in the target cell, while endogenous nucleic acid means a nucleic acid that pre-exists in the genome of the animal. In many embodiments, the sequence of nucleotides present in the exogenous nucleic acid will be one that is not found in the genome of the animal. The subject methods can be used with a variety of animals. Whole animals with which the subject vectors may be employed are generally vertebrates, where in many embodiments the animals are mammals. Of particular interest in many embodiments is the use of the subject vectors to target vertebrate animals, particularly avian and marine animals, e.g., chickens, zebrafish, and the like; mammalian animals, including murine, porcine, ovine, equine, rat, dog, cat, monkey, humans;, and the like.

In the methods of the subject invention, a transposase recognized insertion sequencevector as described above is introduced into or administered to (e.g., injected into, fed to, etc.) a whole animal or directly to specific tissue(s) and/or organ(s) under conditions sufficient for excision of the transposase recognized insertion sequenceflanked nucleic acid from the vector and subsequent integration of the excised nucleic acid into the whole animal genome, as defined above, i.e., into the genomic DNA of a least a portion of the cells making up the whole animal. As the transposase recognized insertion sequencevector is introduced into the animal "under conditions sufficient for excision and integration to occur," the subject method further includes a step of ensuring that the requisite transposase activity is present along with the introduced vector. Depending on the structure of the vector itself, i.e., whether or not the vector includes a region encoding a product having the appropriate transposase activity, the method may further include introducing a second vector into the animal which encodes the requisite transposase activity.

The vector (and second vector where necessary) may be introduced into the animal using any convenient protocol, where the protocol may provide for *in vivo* introduction of the vector. *In vivo* protocols that find use in delivery of the subject vectors include delivery via lipid based, e.g. liposome vehicles, where the lipid based vehicle may be targeted to a specific cell type for cell or tissue specific delivery of the vector. Patents disclosing such methods include: U.S. Patent Nos. 5,877,302; 5,840,710; 5,830,430; and 5,827,703, the disclosures of which are herein incorporated by reference. Other *in vivo* delivery systems may also be employed, including: the use of polylysine based peptides as carriers, which may or may not be modified with targeting moieties, microinjection, electroporation, and the like. (Brooks, A.L, et al. 1998, J. neurosci. Methods V. 80 p: 137-47; Muramatsu, T., Nakamura, A., and H.M. Park 1998, Int. J. Mol. Med. V. 1 p: 55-62). Because of the multitude of different types of vectors and delivery vehicles that may be employed, administration may be by a number of different routes, where representative routes of administration include: oral, topical, intraarterial, intravenous, intraperitoneal, intramuscular, etc. The particular mode of administration depends, at least in part, on the nature of the delivery vehicle employed for the vectors. In many embodiments, the vector or vectors are administered intravascularly, e.g. intraarterially or intravenously, employing an aqueous based delivery vehicle, e.g. a saline solution.

The amount of vector nucleic acid that is introduced into the animal is sufficient to provide for the desired excision and insertion of the exogenous nucleic acid into the genome. As such, the amount of vector nucleic acid introduced should provide for a sufficient amount of transposase activity and a sufficient copy number of the exogenous nucleic acid. The amount of vector nucleic acid that is introduced into the animal varies depending on the efficiency of the particular introduction or transfection protocol that is employed.

Following introduction of the vector DNA into the animal in combination with the requisite transposase, the nucleic acid region of the vector that is flanked by the transposase recognized insertion sequencesof the vector, e.g., the vector nucleic acid positioned between the P element transposase recognized 31 base pair terminal repeats, is excised from the vector via the provided transposase and inserted into the genome of the animal. As such, introduction of the vector DNA into the animal is followed by subsequent transposase mediated excision and insertion of the exogenous nucleic acid carried by the vector into the genome of the animal.

Because of the particular properties of the subject transposon based vectors, the subject vectors may be used to integrate large pieces of DNA into the genome of the whole animal. Generally, the size of DNA that the vectors insert into the genome is at least about 50 bp, usually at least about 1000 bp and more usually at least about 2000 bp, where the size may be as large as 150,000 bp or larger. Where the vector inserts a large piece of DNA into the genome, the size of the inserted DNA ranges from about 30 to 150 kb.

The subject methods of stable integration of exogenous nucleic acid into the genome of a target animal find use in a variety of applications in which the stable integration of an exogenous nucleic acid into the genome of an animal is desired. Applications in which the subject vectors and methods find use include: research applications, polypeptide synthesis applications and therapeutic applications. Each of these representative categories of applications is described separately below in greater detail.

### Research Applications

Examples of research applications in which the subject vectors find use include applications designed to characterize a particular gene. In such applications, the vector is employed to insert a gene of interest into a non-human animal and the resultant effect of the inserted gene on the animal's phenotype is observed. In this manner, information about the gene's activity and the nature of the product encoded thereby can be deduced. The vectors can also be employed to identify and define DNA sequences that control gene expression, e.g. in a temporal (e.g. certain developmental stage) or spatial (e.g. particular cell or tissue type) manner. In such assays, the subject vectors are employed to stably integrate into the genome of a non-human animal a selectable marker gene, e.g. antibiotic resistance, LacZ, etc., where the vector lacks a sufficient promoter for the marker the gene such that the marker is not significantly expressed, if at all, unless it is underneath an endogenous promoter element. If the marker gene is inserted into the non-human animal's genome in sufficient relationship to an endogenous promoter sequence, it will be expressed. From the resultant expression profile of the marker gene, the endogenous promoter that is mediating its expression can then be characterized. Yet another research application in which the subject vectors find use is in the identification and characterization of the results of gene expression studies. For example, a plurality of non-human animals with district integrated vectors are prepared in which the gene of interest is inserted into distinct locations in the genome, where expression of the gene of interest is dependent on endogenous promoter mediation, i.e. where the gene of interest lacks a promoter or is coupled to only a weak promoter. By plurality is meant at least two, where the number usually ranges from about 2 to 20,000, usually from about 2 to 200. This plurality of vector targeted non-human animals may be produced by introducing the vector in a plurality of non-human animals or taking a collection of such pretargeted animals that are homogenous with respect to the insertion site of the gene, i.e. progeny of a single targeted non-human animal, and then introducing transposase into one or more of the constituent members of the collection. The subject vectors can also be used to study integration mutants, where the vector is inserted randomly into the genome and disrupts a random gene whose identity and phenotype can be quickly characterized using standard techniques. One can also employ the subject vectors to produce models in which overexpression and/or misexpression of a gene of interest is produced in a non-human animal and the effects of this mutant expression pattern are observed. One can also use the subject vectors to readily clone genes introduced into a non-human host animal via insertional mutagenesis that yields phenotypes and/or expression patterns of interest. In such applications, the subject vectors are employed to generate insertional mutants through random integration of DNA. The phenotype and/or expression pattern of the resultant mutant is then assayed using any convenient protocol. In those mutants of interest, cloning of the DNA associated with the phenotype and/or expression pattern of interest is readily accomplished through use of the transposase recognized insertion sequencesof the subject vector.

### Polypeptide Synthesis Applications

In addition to the above research applications, the subject vectors also find use in the synthesis of polypeptides, e.g. proteins of interest. In such applications, a vector that includes a gene encoding the polypeptide of interest in combination with requisite and/or desired expression regulatory sequences, e.g. promoters, etc., (i.e. an expression module) is introduced into an appropriate non-human animal to serve as an expression host for expression of the polypeptide (e.g., mouse, chicken, goat, and the like). Following introduction and subsequent stable integration into the non-human animal's genome, the animal is then maintained under any necessary conditions (e.g., inducible system) sufficient for expression of the integrated gene. Once the transformed non-human host expressing the protein is prepared, the protein is then purified to produce the desired protein comprising composition. Any convenient protein purification procedures may be employed, where suitable protein purification methodologies are described in Guide to Protein Purification, (Deuthser ed.) (Academic Press, 1990). For example, the protein could be purified from the milk of a goat with the appropriate attached signal sequences for expression and secretion into the milk. Then a lysate may be prepared from the milk and the protein further purified using HPLC, exclusion chromatography, gel electrophoresis, affinity chromatography, and the like.

### Therapeutic Applications

The subject vectors also find use in therapeutic applications, in which the vectors are employed to stably integrate a therapeutic nucleic acid, e.g. gene, into the genome of a animal, i.e. gene therapy applications. The subject vectors may be used to deliver a wide variety of therapeutic nucleic acids. Therapeutic nucleic acids of interest include genes that replace defective genes in the target animal, such as those responsible for genetic defect based diseased conditions; genes which have therapeutic utility in the treatment of cancer; and the like. Specific therapeutic genes for use in the treatment of genetic defect based disease conditions include genes encoding the following products: factor VIII, factor IX, β-globin, low-density protein receptor, adenosine deaminase, purine nucleotide phosphorylase, sphingomyelinase, glucocerebrosidase, cystic fibrosis transmembrane regulator, α-antitrypsin, CD-18, ornithine transcarbamylase, arginosuccinate synthetase, phenylalanine hydroxylase, branched-chain α-ketoacid dehydrogenase, fumarylacetoacetate hydrolase, glucose 6-phosphatase, α-L-fucosidase, β-glucuronidase, α-L-iduronidase, galactose 1-phosphate uridyltransferase, and the like. Cancer therapeutic genes that may be delivered via the subject vectors include: genes that enhance the antitumor activity of lymphocytes, genes whose expression product enhances the immunogenicity of tumor cells, tumor suppressor genes, toxin genes, suicide genes, multiple-drug resistance genes, antisense sequences, and the like. Because of the length of nucleic acid that can be carried by the subject vectors, the subject vectors can be used to not only introduce a therapeutic gene of interest, but also any expression regulatory elements, such as promoters, and the like, which may be desired so as to obtain the desired temporal and spatial expression of the therapeutic gene.

### KITs

Also provided are kits for use in practicing the subject methods. The subject kits at least include a vector described above having an expression module sufficiently proximal to a transposase recognized insertion sequence, e.g., a P-foot, or a means for making the same, e.g., a provector having restriction endonuclease site, e.g., multiple cloning site, proximal to a transposase recognized insertion sequenceand, optionally, a corresponding restriction endonuclease(s). The subject kits may further include other components that find use in the subject invention, e.g., buffers, delivery vehicles, etc.

The various components of the kit may be present in separate containers or certain compatible components may be precombined into a single container, as desired.

In addition to the above components, the subject kits will further include instructions for practicing the subject invention. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

The following examples are offered by way of illustration and not by way of limitation.

### EXPERIMENTAL

### I. P Element Containing Vectors

Description of vectors used-The nucleic acid vectors used for this experimental section were based wholly or in part (restriction digested and subcloned DNAs) on the pCasper4, pBluescriptII, pcdna3.1, and delta2-3 (or related transposase producing vector). The constructs made were as follows: Restriction enzymes were used to replace the white gene in the pCasper4 vector with the CMV-bgal and SV40-neomycin resistance genes and this new construct will be referred to as C3.1. The next construct was created by removing the CMV-bgal gene from the C3.1 vector and this construct will be referred to as Cneo. Next the bgal gene and SV40 promoter were removed linking the CMV promoter to the neo resistance gene and this construct was called Ccmvneo. Next, the SV40-neo gene was removed from C3.1 and this construct was called Cbgal. Next, the CMV-bgal gene was replaced in the C3.1 vector with pBluescriptII and this construct was called Cbsneo. Finally, pBluescript was added into C3.1, between the CMV promoter and the 3'P foot and this construct was called Cbs3.1.
Name txn units size genes promoters position of promoters

| Vector | Size | Transcriptional Units¹ | Promoters | Genes | Promoter Position |
|---|---|---|---|---|---|
| Casper4 | | 0 | N/A | N/A | N/A |
| C3.1 | | 2 | CMV, SV40 | Bgal, neo^{r} | CMV near P foot |
| Cneo | | 1 | SV40 | neo^{r} | SV40 near P foot |
| Ccmvneo | | 1 | CMV | neo^{r} | CMV near Pfoot |
| Cbgal | | 1 | CMV | Bgal | CMV near P foot |
| Cbsneo | | 1 | SV40 | neo^{r} | None near P foot |
| Cbs3.1 | | 2 | CMV, SV40 | Bgal, neo^{r} | None near P foot |

| | | | | | |
|---|---|---|---|---|---|
| 1- number of transcriptional units that express in vertebrates. A digram of each of the above vectors is provided in Figures 1A to 1G. | | | | | |

### II. Integration in animals is dependent upon concentrations of transposase and P element vector.

A. Strategy: Animals are co-injected with C3.1 and the transposase producing vectors. The injection empoyed is a systemic tail vein injection. Different mice receive different concentrations of each vector in order to determine a concentration that enables the C3.1 vector to integrate into the genome of the mouse. Concurrently, mice are also injected with just the transposase vector at the highest concentration (3 µg) to determine the time course for degredation. After degradation of the transposase producing vector any integrations are stable in the genome of the mouse. Analyses of the genomic DNA from these mice is then made.
B. Experiment: Male mice were injected twice, with a week in between each injection. Two months after the second injection, genomic DNA was harvested from a tail preparation for each mouse and analyzed. PCR assessment never detected the transposase vector. The PCR assessment of the C3.1 vector integrating into the genome occurred in a concentration dependent manner. That is, the more of the vector that integrates or the more of the vector that enables the integration in the injection mixture gave a stronger positive result (more integrations into the genome of the mouse). The results are provided in the following table.

| Mouse Number | Transposase Vector (µg) | C3.1 (µg) | PCR Results (Transposase/C3.1) |
|---|---|---|---|
| 1 | 0.5 | 1 | -/- |
| 2 | 1 | 1 | -/- |
| 3 | 3 | 1 | -/- |
| 4 | 0.5 | 3 | -/- |
| 5 | 1 | 3 | -/- |
| 6 | 3 | 3 | -/+ |
| 7 | 0.5 | 5 | -/+ |
| 8 | 1 | 5 | -/++ |
| 9 | 3 | 5 | -/++ |
| 10 | 0.5 | 10 | -/++ |
| 11 | 1 | 10 | -/++ |
| 12 | 3 | 10 | -/++ |

| | | | |
|---|---|---|---|
| - no band + weak band ++ strong band | | | |

Rats and fish have also been examined for the integration of the C3.1 vector and the results are as seen above, modifying the amount of DNA based on the weight difference between animals.

### III. Integration occurs in every cell type, tissue type and organ examined

Mouse #8 was used as the best positive with the least amount of DNA injected to determine the extent of gene delivery that occurred in the mouse. That is, we harvested a wide variety of tissues from the mouse and extracted the genomic DNA to ascertain the breadth of tissues that could have gene delivery via the C3.1 vector. The C3.1 vector was robustly detected in genomic DNA from every tissue examined, some of which included testis, liver, spleen, heart, lung, brain, intestine. This has been repeated with the same results on female mice.

### IV. Integration can be restricted to a target tissue

Male Mice and rats were injected into their testis. The volume of the injection was reduced to 30 microliters for mice and 100 microliters for rats. These animals gave transgenic offspring but did not show ubiquitous integration in the treated animal. Similar results were obtained localizing the distribution to either the female mammary gland or different sub-cutaneous points of injection. No transgenic offspring were observed from animals injected in a manner not exposing the animal's germ line to the integration vector.

### V. Integrated vectors are heritable

Mice were injected as the #8 mouse in the table above and mated as described in the table below. All litters tested had at least one transgenic offspring. Southern blots and PCR analyses of the offspring indicated that typically 1 to 3 integrations occur per haploid genome for the injected concentration. The integrated DNA has been stable through four generations in mice. Analogous results were generated using rat matings. In addition, targeted injection into the specific organ and tissue type produces non-systemic integration. For example, injection into the testis directly showed heritablity, but no integrations were detected outside of the testis.

| | Mating | | % Transgenic Progeny (N, #independent liters) |
|---|---|---|---|
| Male | Female | Time Post Injection | |
| Injected x2 | No Injection | 2 weeks | 25 (12, 2) |
| Injected x1 | Injected x1 | 1-2days | 40 (20, 4) |
| Injected x1 | Injected x1 | 2 weeks | 38 (13, 2) |
| Injected x1 | Injected x1 | 5 weeks | 71 (14, 2) |
| Injected x1 | Injected x1 | 1 yr. | 44 (9, 2) |
| Control Injected x1 | Control Injected x1 | 1-2days | 0 (19, 3) |

### VI. One actively transcribed gene in proximity to at least one of the P feet is essential for animal integration

The functional elements for integration into animals were examined. The two aspects that were important are that at least transcriptional active promoter is in the vector and that it is located near to a P foot. Though not wishing to be bound by any particular theory, this finding signifies that the DNA binding aspect of the promoter brings transcriptional factors into proximity of the P foot. As transcriptional factors relax the compaction of DNA in order to allow protein access, this may be the key step for transposase excision and integration. That is, any mechanism that relaxes the DNA in proximity to the P foot is likely to enable the transposase to integrate the P element into the genome of an animal.

| Vector | PCR results |
|---|---|
| Casper4 | - |
| C3.1 | + |
| Cneo | + |
| Ccmvneo | + |
| Cbgal | + |
| Cbsneo | - |
| Cbs3.1 | - |

### VII. Genomes from avian, mice, rats, humans do not have sequences that are similar to the P feet

PCR assessment for the prescence and absence of the 5'P foot and 3'P foot were in part used to detect integration of the vector into the genome of animals. During these experiments, it became clear that the hallmark sequences of the P element (the P feet) are not naturally present in non-fruitfly animals as no 5'P or 3'P feet sequences were detected in genomic DNA isolated from birds, mice, rats, and people.

### VIII. Integration of a large DNA fragments into a target cell

P element vectors as described above have the capability to mobilize up to 150 kb of DNA . This presents the opportunity of the P element carrying a DNA sequence with therapeutic use of similar length into a target cell. The manipulation and handling of DNA fragments up to 4 megabases is difficult but has been reproducibly achieved. For example, this vector could allow the potential for analyzing genes on the size of the human huntington gene (∼150 - 200 kb).

### IX. Other transposons are compatible with this method

The beta-galactosidase (BGAL) gene was cloned into the sleeping beauty transposon. To directly compare efficiency of integration with the previous P element transposon results, the P element transposase gene was replaced by the Sleeping Beauty transposase gene (e.g., the same promoter was used to express Sleeping Beauty transposase as was used to express P element transposase). The Sleeping Beauty transposon containing the BGAL gene along with its transposase was injected systemically into the animal using the same criteria as for the P element above (e.g., IP or IV administration, 5 -10 micrograms of transposon DNA, 1-2 micrograms of transposase DNA, etc.). The frequency of integration was less efficient than observed with the P element - one in twenty three progeny were transgenic (4%). However, it has been shown in cells and in vitro that this is also the case. In addition to detecting the germline transmission of the integrated Sleeping Beauty transposon, somatic integration was also detected (e.g., in genomic DNA isolated from liver, intestine and lung tissues of injected animals).

It is evident from the above results and discussion that the subject invention provides a novel nucleic acid vector having a broad range of applications. Advantages of the subject vector over other known nucleic acid vectors include the ability to integrate long stretches of nucleic acid into the genome of a population of target cells of a whole animal, i.e., to integrate the nucleic acid into the genome of a whole animal. This feature is advantageous for a number of reasons, including the ability to integrate an exogenous gene along with its native expression regulatory elements. Another feature of the subject invention is that the vectors provide for random insertion of a foreign nucleic acid, which is desirable in many applications. Yet another advantage of the subject invention is that the subject vectors do not elicit host immune reactions, in contrast with many viral based vectors. In addition, the subject vectors do not carry risk of viral infection or recombination hazards. As such, the subject vectors provide a number of advantages over other vectors currently known and employed in the art, and therefore represent a significant contribution to the art.

## Claims

1. A P-element vector that integrates into the genome of a non-Drosophilidae animal, said vector comprising: a pair of P-element transposase recognized insertion sequences flanking one transcriptionally active gene that is located in sufficient proximity to at least one of the P-element transposase recognised insertion sequences to provide for the animal genome integration, wherein the transcriptionally active gene includes a coding sequence of nucleotides in operational combination with a promoter and optionally further comprising a transposase domain encoding a product having P-element transposase activity wherein said transposase domain is outside of the domain flanked by said pair of P-element transposase recognised insertion sequences.

2. The vector as defined in Claim 1 wherein the transcriptionally active gene comprises nucleic acid endogenous or exogenous to the non-Drosopholidae animal.

3. The use of a P-element vector as defined in Claim 1 or Claim 2 in the manufacture of a composition for insertion of a transcriptionally active gene into the genome of a non-Drosopholidae non-human animal wherein said vector is introduced into said animal under conditions sufficient for transposition to occur so that said transcriptionally active gene is inserted into said genome.

4. The use of a P-element vector as defined in Claim 1 or Claim 2 wherein said transcriptionally active gene is a therapeutic gene in the manufacture of a gene therapy vector for insertion of said therapeutic gene into the genome of a non-Drosopholidae animal wherein said vector is introduced into said animal under conditions sufficient for transposition to occur so that said therapeutic gene is inserted into said genome.

5. The use according to Claim 3 or 4, wherein said vector comprises a transposase domain.

6. The use according to Claim 3 or 4, wherein a second vector is introduced into said animal comprising a transposase domain.

7. The use according to Claim 3 or 4, wherein said target animal is a vertebrate.

8. The use according to Claim 3 or 4, wherein said target animal is a mammalian animal.

9. The use according to Claim 3 wherein said target animal is a rodent.

10. A kit for *in vitro* use in inserting an exogenous nucleic acid into the genome of a target animal cell, said kit comprising:
a P-element vector as defined in Claim 2; and
a nucleic acid sequence encoding a product having P-element transposase activity.

11. A kit for use in inserting an exogenous nucleic acid into the genome of a target non-human animal cell, said kit comprising:
a P-element vector as defined in Claim 2; and
a nucleic acid sequence encoding a product having P-element transposase activity.

12. A kit for use in inserting an exogenous nucleic acid into the genome of a target animal cell, said kit comprising:
a P-element vector as defined in Claim 2 wherein the transcriptionally active gene is a therapeutic gene; and
a nucleic acid sequence encoding a product having P-element transposase activity.

13. A non-Drosophilidae non-human animal having stably integrated into the genome thereof a transcriptionally active gene as defined in Claim 1 or Claim 2 obtainable by the use of any one of Claims 3 to 9 wherein said vector is introduced into a non-Drosopholidae non-human animal, or cells or non-Drosophilidae non-human animals derived therefrom.

## Patentansprüche

1. P-Element-Vektor, der sich in das Genom eines Nicht-Drosophilidae-Tiers integriert, wobei der Vektor Folgendes umfasst: ein Paar von P-Element-Transposase erkannte Insertionssequenzen, die ein transkriptionell aktives Gen flankieren, das in ausreichender Nähe zu zumindest einer der von P-Element-Transposase erkannten Insertionssequenzen liegt, um für die Tiergenom-Integration zu sorgen, worin das transkriptionell aktive Gen eine kodierende Sequenz von Nucleotiden in operationaler Kombination mit einem Promotor umfasst und gegebenenfalls weiters eine Tansposase-Domäne umfasst, die für ein Produkt mit P-Element-Transposase-Aktivität kodiert, worin die Transposase-Domäne außerhalb der Domäne liegt, die von dem Paar von P-Element-Transposase erkannter Insertionssequenzen flankiert werden.

2. Vektor nach Anspruch 1, worin das transkriptionell aktive Gen eine Nucleinsäure umfasst, die für das Nicht-Drosophilidae-Tier endogen oder exogen ist.

3. Verwendung eines P-Element-Vektors nach Anspruch 1 oder 2 zur Herstellung einer Zusammensetzung zur Insertion eines transkriptionell aktiven Gens in das Genom eines Nicht-Drosophilidae-Tiers, das kein Mensch ist, worin der Vektor in das Tier unter Bedingungen eingeführt wird, die ausreichen, damit Transposition erfolgt, so dass das transkriptionell aktive Gen in das Genom insertiert wird.

4. Verwendung eines P-Element-Vektors nach Anspruch 1 oder 2, worin das transkriptionell aktive Gen ein therapeutisches Gen ist, zur Herstellung eines Gentherapie-Vektors zur Insertion des therapeutischen Gens in das Genom eines Nicht-Drosophilidae-Tiers, worin der Vektor in das Tier unter Bedingungen eingeführt wird, die ausreichen, damit Transposition erfolgt, so dass das therapeutische Gen in das Genom insertiert wird.

5. Verwendung nach Anspruch 3 oder 4, worin der Vektor eine Transposase-Domäne umfasst.

6. Verwendung nach Anspruch 3 oder 4, worin ein zweiter Vektor in das Tier eingeführt wird, der eine Transposase-Domäne umfasst.

7. Verwendung nach Anspruch 3 oder 4, worin das Zieltier ein Wirbeltier ist.

8. Verwendung nach Anspruch 3 oder 4, worin das Zieltier ein Säugetier ist.

9. Verwendung nach Anspruch 3, worin das Zieltier ein Nagetier ist.

10. Set zur In-vitro-Verwendung zur Insertion einer exogenen Nucleinsäure in das Genom einer Zieltierzelle, wobei das Set Folgendes umfasst:
einen P-Element-Vektor nach Anspruch 2; sowie
eine Nucleinsäuresequenz, die für ein Produkt mit P-Element-Transposase-Aktivität kodiert.

11. Set zur Verwendung zur Insertion einer exogenen Nucleinsäure in das Genom einer nichtmenschlichen Zieltierzelle, wobei das Set Folgendes umfasst:
einen P-Element-Vektor nach Anspruch 2; sowie
eine Nucleinsäuresequenz, die für ein Produkt mit P-Element-Transposase-Aktivität kodiert.

12. Set zur Verwendung zur Insertion einer exogenen Nucleinsäure in das Genom einer Zieltierzelle, wobei das Set Folgendes umfasst:
einen P-Element-Vektor nach Anspruch 2, worin das transkriptionell aktive Gen ein therapeutisches Gen ist; sowie
eine Nucleinsäuresequenz, die für ein Produkt mit P-Element-Transposase-Aktivität kodiert.

13. Nicht-Drosophilidae-Tier, das kein Mensch ist, mit einem stabil in sein Genom integrierten, transkriptionell aktiven Gen, wie in Anspruch 1 oder Anspruch 2 definiert, das durch eine Verwendung nach einem der Ansprüche 3 bis 9 erhältlich ist, worin der Vektor in ein Nicht-Drosophilidae-Tier, das kein Mensch ist, eingeführt ist, oder davon abstammende Zellen oder Nicht-Drosophilidae-Tiere, die keine Menschen sind.

## Revendications

1. Vecteur d'élément P qui s'intègre dans le génome d'un animal non Drosophilidae, ledit vecteur comprenant: une paire de séquences d'insertion reconnues par une transposase d'élément P flanquant un gène transcriptionnellement actif qui est situé à proximité suffisante d'au moins une des séquences d'insertion reconnues par une transposase d'élément P afin de permettre l'intégration dans le génome animal, où le gène transcriptionnellement actif comprend une séquence de nucléotides codante en association fonctionnelle avec un promoteur et comprenant en outre facultativement un domaine de transposase codant pour un produit ayant une activité de transposase d'élément P, où ledit domaine de transposase est situé en dehors du domaine flanqué par ladite paire de séquences d'insertion reconnues par une transposase d'élément P.

2. Vecteur tel que défini selon la revendication 1, où le gène transcriptionnellement actif comprend un acide nucléique endogène ou exogène pour l'animal non Drosophilidae.

3. Utilisation d'un vecteur d'élément P tel que défini selon la revendication 1 ou la revendication 2, dans la fabrication d'une composition pour l'insertion d'un gène transcriptionnellement actif dans le génome d'un animal non humain non Drosophilidae, où ledit vecteur est introduit dans ledit animal dans des conditions suffisantes pour qu'une transposition puisse avoir lieu de sorte que ledit gène transcriptionnellement actif soit inséré dans ledit génome.

4. Utilisation d'un vecteur d'élément P tel que défini selon la revendication 1 ou la revendication 2, où ledit gène transcriptionnellement actif est un gène thérapeutique, dans la fabrication d'un vecteur de thérapie génique pour une insertion dudit gène thérapeutique dans le génome d'un animal non Drosophilidae, où ledit vecteur est introduit dans ledit animal dans des conditions suffisantes pour qu'une transposition puisse avoir lieu de sorte que ledit gène thérapeutique soit inséré dans ledit génome.

5. Utilisation selon la revendication 3 ou 4, où ledit vecteur comprend un domaine de transposase.

6. Utilisation selon la revendication 3 ou 4, où un second vecteur est introduit dans ledit animal comprenant un domaine de transposase.

7. Utilisation selon la revendication 3 ou 4, où ledit animal cible est un vertébré.

8. Utilisation selon la revendication 3 ou 4, où ledit animal cible est un animal mammifère.

9. Utilisation selon la revendication 3, où ledit animal cible est un rongeur.

10. Kit destiné à être utilisé *in vitro* afin d'insérer un acide nucléique exogène dans le génome d'une cellule animale cible, ledit kit comprenant:
un vecteur d'élément P tel que défini selon la revendication 2; et
une séquence d'acide nucléique codant pour un produit ayant une activité de transposase d'élément P.

11. Kit destiné à être utilisé pour insérer un acide nucléique exogène dans le génome d'une cellule animale non humaine cible, ledit kit comprenant:
un vecteur d'élément P tel que défini selon la revendication 2; et
une séquence d'acide nucléique codant pour un produit ayant une activité de transposase d'élément P.

12. Kit destiné à être utilisé pour insérer un acide nucléique exogène dans le génome d'une cellule animale cible, ledit kit comprenant:
un vecteur d'élément P tel que défini selon la revendication 2, où le gène transcriptionnellement actif est un gène thérapeutique; et
une séquence d'acide nucléique codant pour un produit ayant une activité de transposase d'élément P.

13. Animal non humain non Drosophilidae ayant stablement intégré dans son génome un gène transcriptionnellement actif tel que défini selon la revendication 1 ou la revendication 2, pouvant être obtenu par l'utilisation de l'une quelconque des revendications 3 à 9, où ledit vecteur est introduit dans un animal non humain non Drosophilidae, ou dans des cellules ou des animaux non humains non Drosophilidae dérivé(e)s de celui-ci.
